# DEMANDE DE BREVET EUROPEEN

(11) **EP 0 723 002 A1**
(43) Date de publication de la demande: **24.07.1996**
(21) Numéro de dépôt: 96400034.3
(22) Date de dépôt: 08.01.1996
(51) Int. Cl.: C10M 135/04, B01J 37/20, C07C 319/00

(54) **Hydrocarbures éthyléniques sulfurés par le soufre élémentaire en présence de carbonate ou d'hydrogénocarbonate alcalin, leur préparation et leurs utilisations**

(30) Priorité: 10.01.1995 FR 9500282
(71) Demandeur: INSTITUT FRANCAIS DU PETROLE, F-92502 Rueil-Malmaison (FR)
(72) Inventeur: Lacome, Thierry, F-92500 Rueil Malmaison (FR); Delfort, Bruno, F-75005 Paris (FR); Born, Maurice, F-92000 Nanterre (FR)
(74) Mandataire: Andreeff, François

(57) **Abrégé**

On décrit des produits soufrés dérivant d'hydrocarbures mono ou polyéthyléniques par sulfuration au moyen de soufre élémentaire en présence de carbonate d'hydrogénocarbonate de métal alcalin et en présence d'eau et/ou d'au moins un alkylèneglycol.

Les produits soufrés, qui peuvent contenir de 20 à 70 % en masse de soufre, sont généralement solubles dans les lubrifiants, dont ils améliorent les propriétés antiusure et extrême-pression. lls peuvent également être utilisés comme agents de sulfuration notamment dans la préparation de catalyseurs de raffinage de produits pétroliers.

## Description

L'invention concerne de nouveaux produits organiques soufrés, un procédé pour leur préparation et leurs utilisations.

Divers produits organiques soufrés sont utilisés depuis longtemps comme additifs dans les lubrifiants pour en améliorer notamment les propriétés antiusure et extrême-pression. Il peut s'agir notamment de lubrifiants pour moteurs, pour engrenages fortement chargés ou de lubrifiants pour le travail des métaux.

Comme additifs antiusure et extrême-pression, ont été largement décrits des produits organiques soufrés obtenus par divers procédés comprenant principalement deux étapes :
1) la formation d'un produit d'addition entre un chlorure de soufre (mono ou dichlorure de soufre) et un composé à insaturation éthylénique, et
2) la réaction de ce produit d'addition avec un composé sulfuré: sulfure, hydrosulfure ou polysulfure de métal alcalin (par exemple le sodium), mis en jeu tels quels ou formés in situ par réaction d'hydrogène sulfuré (H₂S) ou de mercaptans avec un hydroxyde de métal alcalin (par exemple la soude).

Pour accroître la teneur en soufre des produits obtenus il était possible de mettre en jeu du soufre élémentaire au cours de la seconde étape décrite ci-dessus.

Du fait de la mise en jeu de composés chlorés dans la première étape de leur préparation, les produits soufrés de ce type contiennent toujours une certaine quantité résiduelle de chlore, qui a pu être réduite à moins de 250 ppm par diverses améliorations apportées au procédé, notamment par la demanderesse, ces dernières années. De plus, ces procédés engendrent la formation, comme sous produits, de chlorure de métal alcalin (en général NaCl) et de dérivés organiques alcalins hydrosolubles, que l'on retrouve sous la forme d'une solution aqueuse très polluante, produite en quantité très importante et, de ce fait, très coûteuse à éliminer.

On connaît par ailleurs divers procédés de préparation de produits soufrés ne mettant pas en jeu de composés chlorés. Certains de ces procédés sont basés sur la sulfuration des oléfines par le soufre élémentaire et l'hydrogène sulfuré (H₂S). Ces procédés présentent l'inconvénient de mettre en jeu un réactif extrêmement toxique, difficile à stocker et à transporter et de développer des pressions généralement élevées qui peuvent aller par exemple jusqu'à 90 bars.

D'autres procédés mettent en jeu du soufre élémentaire et des hydrosulfures alcalins ou alcalino-terreux.

On a maintenant découvert qu'il était possible de préparer des produits soufrés à partir d'hydrocarbures mono ou polyéthyléniques en utilisant du soufre élémentaire comme seul réactif soufré, en opérant en présence d'au moins un carbonate ou hydrogénocarbonate de métal alcalin et en présence d'eau en une proportion de 50 à 500 cm³ par insaturation éthylénique de l'hydrocarbure de départ et/ou d'au moins un alkyléneglycol.

Les produits soufrés de l'invention sont exempts de chlore, ils présentent en général une teneur en soufre pouvant aller d'environ 20 à 70% en masse selon l'hydrocarbure éthylénique de départ. Les produits soufrés de l'invention sont solubles à des degrés divers dans les huiles minérales et dans les polyalphaoléfines hydrogénées, même - ce qui est tout à fait inattendu - les produits ayant une teneur en soufre élevée.

Les produits soufrés de l'invention peuvent être définis d'une manière générale par le fait qu'ils sont obtenus par réaction d'au moins un hydrocarbure mono ou polyéthylénique renfermant en général de 2 à 36 atomes de carbone et de 1 à 3 insaturations éthyléniques avec une quantité appropriée de soufre élémentaire en présence d'au moins un un carbonate ou hydrogénocarbonate de métal alcalin et en présence d'eau et/ou d'au moins un alkyléneglycol.

Les hydrocarbures mono ou polyéthyléniques de départ peuvent être à chaîne ouverte, linéaire ou ramifiée, ou cycliques. Comme exemples, on peut citer : l'éthylène, le propylène, le 1-butène, les n-butènes, l'isobutène, les divers pentènes, les divers hexènes (par exemple le 2, 3 -diméthyl 1- butène) ainsi que le 1, 3-butadiène, l'isoprène, le cyclopentadiène, le dicyclopentadiène, les dimères et trimères de l'isobutène, les trimères et tétramères du propylène ainsi que les polyalphaoléfines non hydrogénées de faible masse molaire (allant par exemple jusqu'à environ 500). On utilise de préférence l'isobutène.

Dans la réaction de préparation des produits soufrés de l'invention, on met en jeu une proportion de soufre élémentaire allant jusqu'à environ 250 g (soit environ 8 moles) par insaturation éthylénique dans l'hydrocarbure de départ.

On introduit également dans le milieu de réaction de l'eau en une proportion de 50 à 500 cm³ par insaturation éthylènique de l'hydrocarbure de départ et/ou un alkylèneglycol, tel que l'éthylèneglycol ou le propylèneglycol, en une proportion pouvant varier par exemple de 10 à 150 g par insaturation éthylénique de l'hydrocarbure de départ.

Comme carbonates et hydrogénocarbonates de métal alcalin, on considère plus particulièrement dans l'invention ceux de lithium, de sodium et de potassium. On utilise le plus souvent ceux de sodium ou de potassium. La proportion de carbonate ou d'hydrogénocarbonate mise en jeu peut aller par exemple de 3 .10⁻³ à 1,2 mole par insaturation éthylénique de l'hydrocarbure de départ.

On remarque avec surprise que le carbonate ou l'hydrogénocarbonate de métal alcalin peut être utilisé notamment en très faible proportion (quasi catalytique) et que les produits soufrés finalement obtenus ne renferment pas de métaux alcalins ou alcalino-terreux et ne présentent aucun caractère basique. Ainsi, on peut penser que le carbonate ou l'hydrogénocarbonate agit essentiellement comme promoteur de la réaction de sulfuration.

En général, la réaction est réalisée à une température de 50 à 200°C et le plus souvent de 90 à 160°C.

La pression, qui dépend de manière prépondérante de la nature du ou des hydrocarbures mono ou polyéthyléniques de départ, peut aller de la pression atmosphérique jusqu'à par exemple 50 bars. Ainsi, pour les hydrocarbures éthyléniques gazeux dans les conditions normales, la pression s'établit entre 10 et 50 bars selon le proportion entre l'hydrocarbure éthylénique et le carbonate ou l'hydrogénocarbonate de métal alcalin mis en jeu. La durée de la réaction est par exemple de 0,5 à 24 heures .

Les produits de l'invention sont des produits liquides renfermant généralement de 20 à 70 % en masse de soufre ; ils sont limpides et homogènes même aux hautes teneurs en soufre. Leur coloration varie selon la nature de l'hydrocarbure de départ et la quantité de soufre fixée. Ils peuvent être de très peu ou peu colorés (c'est la cas par exemple pour les hydrocarbures monoéthyléniques tels que l'isobutène) à très colorés (dans le cas par exemple des hydrocarbures polyéthyléniques).

Dans le cas des hydrocarbures monoéthyléniques, les produits soufrés se caractérisent par la quasi absence d'atomes de carbone éthyléniques.

Les teneurs en soufre des produits de l'invention leur confèrent des propriétés antiusure et extrême - pression telles qu'ils peuvent utilisés avantageusement comme additifs dans les huiles et graisses lubrifiantes, notamment dans la formulation d'huiles pour engrenages d'automobiles et d'huiles industrielles.

Pour ces utilisations, on incorpore en général les produits de l'invention aux compositions lubrifiantes à des concentrations de 0,05 à 20 %, de préférence de 0,5 à 10 % en masse.

Certains des produits soufrés de l'invention, notamment ceux qui dérivent de la sulfuration de l'isobutène, peuvent encore être utilisés comme agents de sulfuration en particulier dans la préparation de catalyseurs de raffinage de produits pétroliers.

Les exemples suivants illustrent l'invention.

### Exemple 1

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 mole) de soufre, 22,5g (0,21 mole) de carbonate de sodium, 202 g d'eau et 36g (0,642 mole) d'isobutène. On porte le milieu à la température de 130°C pendant 10heures. La pression maximale atteinte est de 24 bars. Après retour à la température ambiante, on extrait le milieu avec 150 ml de n-heptane. Après lavage à la soude à 10% en masse, puis lavage à l'eau, on évapore le n-heptane sous pression réduite et on recueille 81g d'un liquide limpide dont la teneur en soufre est de 63,5% en masse.

### Exemple 2

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 mole) de soufre, 22,5g (0,163 mole) de carbonate de potassium, 202g d'eau et 36g (0,642 mole) d'isobutène. On porte le milieu à la température de 130°C pendant 10 heures. La pression maximale atteinte est de 26 bars. Après retour à la température ambiante, on extrait le milieu avec 150 ml de n-heptane. Après lavage à la soude à 10% en masse, puis lavage à l'eau, on évapore le n-heptane sous pression réduite et on recueille 95,3g d'un liquide limpide dont la teneur en soufre est de 67 % en masse.

### Exemple 3

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 mole) de soufre, 22,5 g (0,416 mole) d'hydrogénocarbonate de sodium, 202 g d'eau et 36g (0,642 mole) d'isobutène. On porte le milieu à la température de 130°C pendant 10 heures. La pression maximale atteinte est de 25 bars. Après retour à la température ambiante, on extrait le milieu avec 150 ml de tn-heptane Après lavage à la soude à 10% en masse, puis lavage à l'eau, on évapore le n-heptane sous pression réduite et on recueille 71 g d'un liquide limpide dont la teneur en soufre est de 61,3 % en masse.

### Exemple 4

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 50 g (1,56 mole) de soufre, 15,0 g (0,15 mole) d'hydrogénocarbonate de potassium, 135g d'eau et 72g (1,285 mole) d'isobutène. On porte le milieu à la température de 130°C pendant 10 heures. La pression maximale atteinte est de 36 bars. Après retour à la température ambiante, on extrait le milieu avec 150 ml de n-heptane. Après lavage à la soude à 10% en masse, puis lavage à l'eau, on évapore le n-heptane sous pression réduite et on recueille 70,5 g d'un liquide limpide dont la teneur en soufre est de 64,6 % en masse.

### Exemple 5

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5 g (3,51 mole) de soufre, 1,2g (0,0087 mole) de carbonate de potassium, 225g d'eau et 36g (0,642 mole) d'isobutène. On porte le milieu à la température de 130°C pendant 10 heures. La pression maximale atteinte est de 25 bars. Après retour à la température ambiante, on extrait le milieu avec 150 ml de n-heptane. Après lavage à la soude à 10% en masse, puis lavage à l'eau, on évapore le n-heptane sous pression réduite et on recueille 16 g d'un liquide limpide dont la teneur en soufre est de 41 % en masse.

### Exemple 6

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 mole) de soufre, 15g (0,375 mole) de carbonate de sodium, 135g d'eau, 44,8g (0,77 mole) d'isobutène et 2,6g (0,046 mole) de 1,3-butadiène. On porte le milieu à la température de 130°C pendant 12 heures. La pression maximale atteinte est de 29 bars. Après retour à la température ambiante, on extrait le milieu avec 150 ml de n-heptane. Après lavage à la soude à 10% en masse, puis lavage à l'eau, on évapore le n-heptane sous pression réduite et on recueille 78 g d'un liquide limpide dont la teneur en soufre est de 62 % en masse.

### Exemple 7

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 mole) de soufre, 22,5g (0,212 mole) de carbonate de sodium, 202g d'eau, 36g (0,32 mole) de diisobutène. On porte le milieu à la température de 130°C pendant 10 heures. La pression maximale atteinte est de 13 bars. Après retour à la température ambiante, on extrait le milieu avec 150 ml de n-heptane. Après lavage à la soude à 10% en masse, puis lavage à l'eau, on évapore le n-heptane sous pression réduite et on recueille 33 g d'un liquide limpide dont la teneur en soufre est de 31 % en masse.

L'examen par C13 RMN et par analyse élémentaire des produits préparés dans les exemples de 1 à 7 indique l'absence d'atomes de carbone éthyléniques ainsi que de sodium ou de potassium pour tous les produits des exemples de 1 à 7.

### Exemple 8: Examen de la solubilité des produits de l'invention

Les produits de l'invention préparés comme décrits dans les exemples 1 à 7 sont examinés pour leur solubilité dans une huile minérale 130 Neutral Solvent et une huile synthétique constituée d'une polyalphaoléfine hydrogénée (PAO 6) à une concentration permettant d'ajuster la teneur en soufre à 2% en masse. Les résultats sont rassemblés dans le tableau I.

### Exemple 9: Caractérisation des propriétés extrême-pression

Les produits de l'invention préparés comme décrits dans les exemples 1, 2, 3,4 et 6 ci-dessus sont caractérisés pour leurs propriétés extrême-pression dans une huile minérale 130 Neutral Solvent à une concentration permettant d'ajuster la concentration en soufre due à l'additif à 2% en masse. La caractérisation est effectuée au moyen d'une machine 4 billes selon l'essai ASTM D2783. Les résultats sont rassemblés dans le Tableau II.

**TABLEAU I**

| **PRODUIT DE L'EXEMPLE N°** | % en masse **ADDITIF/HUILE** | % en masse **S/HUILE** | **SOLUBILITE/HUILE MINERALE130NS** | **SOLUBILITE/HUILE SYNTHETIQUE PAO 6** |
|---|---|---|---|---|
| 1 | 3,06 | 2 | oui | oui |
| 2 | 2,99 | 2 | oui | oui |
| 3 | 3,26 | 2 | oui | oui |
| 4 | 3,10 | 2 | oui | - |
| 5 | 4,88 | 2 | oui | oui |
| 6 | 3,23 | 2 | oui | - |
| 7 | 6,45 | 2 | oui | oui |

**TABLEAU II**

| PRODUIT DE L'EXEMPLE N° | % en masse ADDITIF/HUILE | % en masse S/HUILE | Essais sur machine 4 billes | |
|---|---|---|---|---|
| | | | Charge de soudure (daN) | Indice charge/usure (daN) |
| 1 | 3,06 | 2 | 380 | 74 |
| 2 | 2,99 | 2 | 400 | 80 |
| 3 | 3,26 | 2 | 440 | 84 |
| 4 | 3,10 | 2 | 420 | 81 |
| 6 | 3,23 | 2 | 420 | 80 |

## Revendications

1. Produit soufré caractérisé en ce qu'il est obtenu par réaction d'au moins un hydrocarbure mono ou polyéthylènique renfermant de 2 à 36 atomes de carbone et de 1 à 3 insaturations éthyléniques avec du soufre élémentaire en présence d'au moins un carbonate ou hydrogénocarbonate de métal alcalin et en présence d'eau en une proportion de 50 à 500 cm3 par insaturation éthylénique de l'hydrocarbure de départ et/ou d'au moins un alkylèneglycol.

2. Produit soufré selon la revendication 1, caractérisé en ce que l'hydrocarbure mono ou polyéthylénique est choisi parmi l'éthylène, le propylène, le butène-1, les n-butènes-2, l'isobutène, les divers pentènes, les divers héxènes ainsi que le 1, 3-butadiène, l'isoprène, le cyclopentadiène, le dicyclopentadiène les dimères et trimères de l'isobutène, les trimères et tétramères du propylène, ainsi que les polyalphaoléfines non hydrogénées de masse molaire allant jusqu'à environ 500.

3. Produit soufré selon la revendication 1 ou 2, caractérisé en ce que ledit hydrocarbure est l'isobutène.

4. Produit soufré selon l'une des revendications 1 à 3, caractérisé en ce que l'on met en jeu une proportion de soufre élémentaire allant jusqu'à environ 250 g (soit environ 8 moles) par insaturation éthylénique de l'hydrocarbure de départ.

5. Produit soufré selon l'une des revendications 1 à 4, caractérisé en ce que l'alkylènyglycol est l'éthylèneglycol ou le propylèneglycol.

6. Procédé selon l'une des revendications 1 à 5, caractérisé en ce que ledit alkylèneglycol est mis en jeu en une proportion de 10 à 150 g par insaturation éthylénique de l'hydrocarbure de départ.

7. Produit soufré selon l'une des revendications 1 à 6, caractérisé en ce que le carbonate ou l'hydrogénocarbonate de métal alcalin est choisi parmi ceux de lithium, de sodium et de potassium et est mis en jeu en une proportion de 3.10⁻³ à 1,2 mole par insaturation éthylénique de l'hydrocarbure de départ.

8. Produit soufré selon l'une des revendications 1 à 7, caractérisé en ce que la réaction est réalisée à une température de 50 à 200°C et sous une pression allant de la pression atmosphérique jusqu'à 50 bars.

9. Produit soufré selon l'une des revendications 1 à 8 caractérisé en ce qu'il renferme de 20 à 70% en masse de soufre.

10. Utilisation d'un produit soufré selon des revendications 1 à 9 comme additif dans des compositions lubrifiantes, à une concentration de 0,05 à 20% en masse.

11. Utilisation d'un produit soufré selon des revendications 1 à 9 comme agent de sulfuration dans la préparation de catalyseurs de raffinage de produits pétroliers.
